# EUROPEAN PATENT APPLICATION

(11) **EP 4 116 986 A1**
(43) Date of publication of application: **11.01.2023**
(21) Application number: 21184384.2
(22) Date of filing: 07.07.2021
(51) Int. Cl.: G16H 20/10, G16H 50/20

(54) **SYSTEMS AND METHODS FOR OPTIMIZATION OF DISEASE THERAPY**

(71) Applicant: Fresenius Medical Care Deutschland GmbH, 61352 Bad Homburg (DE)
(72) Inventor: NERI, Luca, 20144 Milano (MI) (IT); BELLOCCHIO, Francesco, 27010 Siziano (PV) (IT); BARBIERI, Carlo, 26013 Crema (IT)
(74) Representative: Dreyhsig, Jörg

(57) **Abstract**

A therapy optimization system for supporting therapy of diseases, particularly Mineral Bone Disorder in Chronic Kidney Disease, is provided. The system comprises interfacing modules for exchanging therapy-related data with several subsystems of a clinical decision-support system, and a therapy-support module for controlling the data exchange via the interfacing module, whereby the therapy-related data is processed by the therapy-support module to generate and to additionally transfer therapy recommendations and/or data-input requests via the interfacing modules. Furthermore, a computer-implemented method for optimization of disease therapy, a computer program, and a computer program product that, when executed on a computing system, cause the system to perform the steps of the method are provided.

## Description

### Technical Area

The present invention relates to, in general, the therapy of diseases, especially the therapy of Mineral Bone Disorder in Chronic Kidney Disease, and, particularly, to a therapy optimization system for optimizing treatment outcomes, and a method and computer program (product) to control the system.

### Background

Medical treatment of chronic kidney disease - mineral bone disorder (CKD-MBD) is one of the extremely challenging clinical problems for patients with kidney disease. CKD-MBD is a systemic disorder associated with increased cardiovascular morbidity, bone fractures and mortality risk. It is a common complication of advanced renal disease but when effectively treated, through the normalization of the biomarkers related to mineral bone metabolism, the benefits in terms of longer survival and lower rate of cardiovascular events, have been reported in different clinical studies. The condition is characterized by a systemic disorder of mineral and bone metabolism generally manifested as abnormal levels of serum phosphate, serum calcium, parathyroid hormone (PTH) and serum vitamin D. There are several different clinical interventions (e.g., drugs, diet, parotidectomy, etc.) to manage and treat this disorder but as these biomarkers characterizing the condition are highly interdependent and behave differently through different physiological pathways, correcting them and maintaining the patient with normal levels at the same time, poses a general clinical challenge.

Furthermore, patient behavioral patterns strongly influence the disorder. Although there are several guidelines on the use of the drugs related to CKD-MBD, the efficacy of the treatment can be hampered by the uncertainty about patient's behavior. The possibility of adapting the drugs dose based on real-time monitoring of patient behavior, sensors' measurements, patient's symptoms, etc. might significantly improve patient's outcomes.

A complex pathophysiology, deviations from medication adherence and their effects, and significant contributions to the outcomes by aspects related to diet and physical activity are the major problems the clinicians must face for an effective treatment.

The clinical challenges in the therapy of CKD-MBD can be transferred to the therapy of other diseases, in which patient's behavior and medication adherence significantly influence the therapy outcomes, e.g. hypotension, diabetes, coronary heart/artery disease, asthma, obesity, cancer, and others.

Clinical Decision Support Systems (CDSS) have been applied for different problems in medicine. The growing availability of medical data and the advances in computer-implemented data evaluation are two of the major drivers for the development and application of such digital solutions within CDSS. CDSS can be very effective in supporting clinicians and other involved parties in the treatment and management of the patients' disorders, especially for diseases influenced by many intercorrelated factors such as CKD-MBD.

The treatment of diseases, particularly of CKD-MBD, can be optimized by exploiting advantages of technical aspects of a CDSS tailored to the underlying medical problem.

### Summary of the invention

To address the stated challenges about therapy of diseases, particularly CKD-MBD, it is of utmost importance to provide a system able to analyze, in real-time, the specificity of the patient's condition, in terms of all medically contributing aspects, and adapt the management of the therapy consequently through a specific program of clinical, behavioral, and educational intervention and interactions between clinician and patient. The disclosed system relates to the consideration of the multi-factorial and complex medical nature of specific diseases, particularly CKD-MBD, and underlying physiology, and to the combination of a set of subsystems to analyze and manage the subproblems concurring with this disorder. The subsystems are focused on specific aspects of the disease, particularly CKD-MBD, (e.g., medication adherence, patient's dose-response relation, diet, physical activity, clinical intervention, etc.) and the central element of the system combines and processes the different subsystems' inputs and outputs in order to optimize a general strategy of treatment.

In the following, the description will be focused on the therapy of CKD-MBD. The transferability of the disclosed aspects of the invention to other diseases, in which the patient's behavior and medication adherence significantly influence the therapy outcomes, e.g. hypotension, diabetes, coronary heart/artery disease, asthma, obesity, cancer, and others, is preserved and the application of the invention to those diseases is comprised in the scope of the present invention.

The present system relates to supporting the therapy of CKD-MBD by personalized and real-time adaptive care interactions, which represents a holistic approach to the CKD-MBD patient. For this, a digital ecosystem composed of a set of subsystems tackling the different subproblems of the medical condition are employed. The substantial element of the present system is the therapy optimization system, a therapy optimizer system, at the core of the ecosystem. The therapy optimizer system coordinates and combines the different system parts with the aim of optimizing the therapy in terms of interventions and interactions.

The whole CDSS adapted to represent CKD-MBD therapy support consists of a set of interactive subsystems.

One first subsystem of the CDSS, a patient monitor, can be employed for monitoring and analyzing the medical patient status and behavior: medication adherence, patient activity, diet, self-assessment through sensors and electronic patient-reported outcomes. This part can interact with the patient through a user interface to collect specific information (e.g., by questionnaire), to give recommendation (e.g., drug dose adjustment) and to establish interaction with clinicians (e.g. remote communication).

One second subsystem of the CDSS, a dose-response simulator, can be employed for simulating drug-dose response of a patient with certain medical status. For each CKD-MBD related drug (e.g., vitamin D, phosphate binder, etc.) and by considering the specificity of the patient (e.g., biomarkers, comorbidities, lifestyle, medications, etc.), a dose-response relation can be computed.

One third subsystem of the CDSS, a clinical monitor, can be employed for patent monitoring and for evaluating clinical intervention adjustment. This subsystem can interact with the clinician through a user interface representing the general status of the patient, drugs' prescription, medication adherence, any other information useful for assessing the CKD-MBD therapy and for interacting with the patient (e.g., requesting a visit, remote communication, etc.).

The core of the CDSS is the CKD-MBD therapy optimizer subsystem. It can coordinate and combine the information transmitted from the subsystems to generate recommendations for the patient and clinicians. Furthermore, it can manage the inputs/outputs of each subsystem and the communication between patient and clinician.

The underlying problem is addressed by the independent claims 1 and 13 of the present invention.

According the invention, a therapy optimizer system for supporting the therapy of diseases, particularly CKD-MBD, is provided. It comprises interfacing modules configured for exchanging therapy-related data with the subsystems of a CDSS adapted to the, particularly CKD-MBD, therapy, which are one or more of a clinical data base, a clinical monitor, a dose-response simulator and/or a patient monitor. It further comprises a therapy-support module configured to control the exchange of therapy-related data by the interfacing modules. The therapy-support module is further configured to process the transmitted therapy-related data to generate therapy-related recommendations to be additionally transmitted by the interfacing modules.

According to the invention, a computer-implemented method is provided for supporting the therapy of diseases comprising the steps transmitting therapy-related data to interfacing modules configured for transmitting therapy-related data with one or more of a clinical data base, a clinical monitor, a dose-response simulator, a patient monitor; analyzing the therapy-related data transmitted by the interfacing modules with respect to their sufficiency for producing therapy-related recommendations; Generating, based on the sufficiency analysis, data-input requests to be transmitted by the respective interfacing modules; Generating, based on the therapy-related data transmitted to the interfacing modules, therapy-related recommendations to be transmitted by the respective interfacing modules; and controlling the interfacing modules to transmit respective data-input requests and/or therapy-related recommendations.

The dependent claims 2-12, 14, and 15 are directed to preferred embodiments of the present invention.

### Description of the drawings

In the following, preferred embodiments of the present invention will be explained with reference to the accompanying figures:
Fig. 1: a schematic depicting the components of the CDSS adapted to the therapy of e.g. CKD-MBD
Fig. 2: a schematic depicting the components of the therapy optimizer subsystem of the CDSS according to the present invention
Fig. 3: a schematic depicting data exchanged between the therapy optimizer system and other subsystems of the CDSS according to the present invention
Fig. 4: a flowchart depicting an example process performed by the therapy optimizer system according to the present invention

### Detailed description of the embodiments

Specific embodiments of the present disclosure will be described below.

The present invention provides a system, a method, and a computer program (product) to optimize therapy of CKD-MBD by coordinating the exchange of health-related data between different interfacing subsystems of a CDSS adapted to support the therapy of CKD-MBD.

The system further includes the processing of health-related data exchanged to generate, depending on the data exchanged, recommendations to either directly affect the contribution of a sub-system for the purpose of improved therapy outcome when implemented, and/or requests additional input from the users of the respective sub-systems of the CDSS.

Furthermore, the system adaptively provides current medical status and potential medical interactions with respect to optimize therapy of CKD-MBD to involved patients and clinicians, and therefore facilitates therapy-supporting communication.

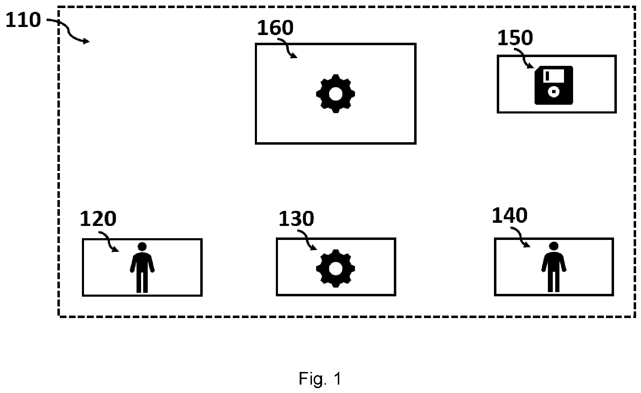

**Fig. 1** provides a schematic depicting the components of the CDSS adapted to the therapy of e. g. CKD-MBD.

The Clinical Decision Support System (CDSS), shown as 110, comprises a multitude of subsystems (120, 130, 140, 150).

The number of subsystems and their respective functions are adapted to the medical condition(s), for which a clinical decision is to be supported. In a general, less specialized CDSS, i.e. a CDSS supporting decisions on a variety of clinical conditions, more subsystems can obviously be included.

No limitation to spatial allocation of the subsystems is to be deduced from the depiction since data communication between the subsystems can be established by well-known remote data-transfer means. All subsystem can also be implemented as software systems on generic or specialized computational hardware. Allocation of several subsystem functions to a shared pool of computational hardware and merging subsystems are well known aspects of computation in a distributed "cloud" environment which are well applicable here.

The support system disclosed here is adapted to the therapy of a specific disease, particularly CKD-MBD. For this, the subsystems are adapted to reflect the aspects influencing the therapeutic outcome of the specific condition, particularly CKD-MBD, and the monitoring of their status. For this purpose, the system can comprise a patient monitor (120), a clinical monitor (140), a dose-response simulator (130), a clinical data base (150), and the therapy optimizer system (160) for CKD-MBD of the present invention which is provided with means for data communication to each of the other subsystems.

The patient monitor (120) can be any system comprising sensors, software, other means and the like able to collect information about the therapy-related status (symptoms, physiological measurement, etc.) and the behavior of the patient (medication adherence, diet, physical activity, etc.) from the patient. The interaction between patient and patient monitor can be conducted via one of many known means for user interfaces (e.g. touch-sensitive display) and the system ca comprise means for data exchange (e.g. remotely via internet). Example embodiments could be a usual smartphone or a usual notebook computer, optionally connected with additional devices for physiological measurement (sensors for measurements of blood pressure, weight, etc.).

The clinical monitor (140) can be any system comprising sensors, software, other means and the like able to represent a set of information about the patient, their health status and their behavior (e.g., physiological measurements, symptoms, comorbidities, diet, drugs taken, previous dose-response measurements, etc.) to a clinician or other medically versed person. The system can comprise a warning system that notifies the user about medical situations that require clinician attention (e.g., dangerous levels of some biomarkers) and at least one communication channel (text, voice, video, ...) to interact with the patient synchronously or asynchronously. The interaction between clinician and clinical monitor can be conducted via known means for user interfaces (e.g. touch-sensitive display) and the system comprises means for data exchange (e.g. remotely via internet). Example embodiments could be a usual smartphone or a usual notebook computer. The dose-response simulator (130) can be any system comprising sensors, software, other means and the like able to perform simulations about the relation between a class of drugs (for CKD-MBD e.g. vitamin D, phosphate binders, etc.) and some therapy-related biomarkers (serum calcium, phosphate, PTH, etc.). The simulator can include for the simulation, for a more accurate assessment, a set of information related to the patient and his behavior (physiological measurements, comorbidities, diet, drugs taken, previous dose-response measurements, etc.). The simulator can employ several known models for simulating pharmacokinetics and physiological dynamics based on input of therapy-related data to simulate the dynamics of therapy-related patient biomarkers in dependence of the drugs and their respective dosing.

The clinical data base (150) can be any system comprising means for data storage to allow storage of electronic medical records related to patient health.

The subsystems of the CDSS (120, 130, 140, 150) separately are well known in the state of the art in various forms of embodiments Their selection from various other subsystems potentially employed in a general CDSS, and their specific combination allow to technically represent the medical aspects contributing to the outcomes specific for the therapy of CKD-MBD and the condition's specific interdependence of therapeutic aspects.

The core component of the CDSS, the therapy optimizer system (160 can technically adapt a generic CDSS to address the specific underlying processes involved in the therapy of CKD-MBD by controlling and coordinating the subsystems and by processing the data exchanged with them to improve CKD-MBD therapy outcomes.

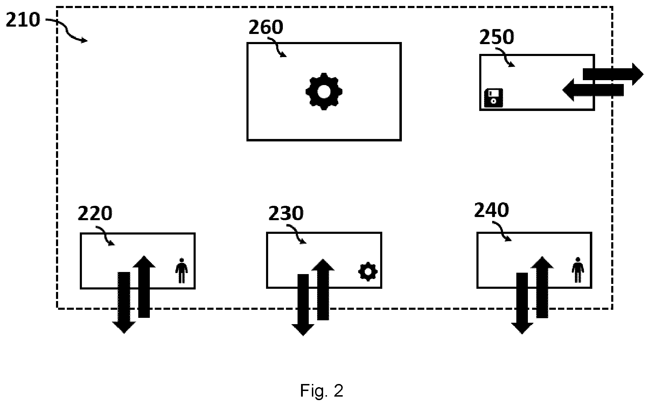

**Fig. 2** provides a schematic depicting the components of the therapy optimizer subsystem of the CDSS according to the present invention.

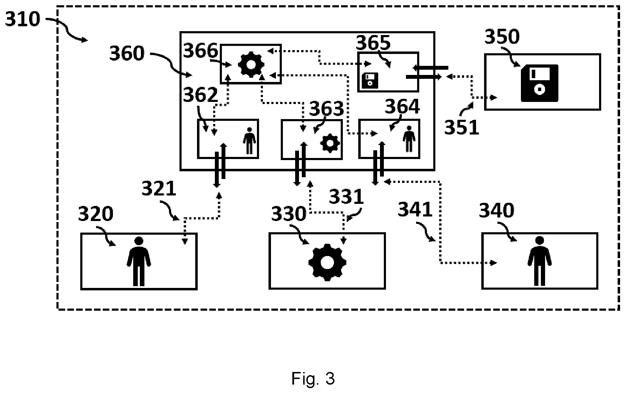

The therapy optimizer system (210) of the present invention, referenced by (160) in fig. 1 and by (360) in fig. 3, comprises a multitude of modules (220, 230, 240, 250) for interfacing with subsystems of a CDSS adapted to support the therapy of CKD-MBD and a therapy-support module (260) for the coordination and control of the interfacing modules.

The selection and synergetic combination of the specific interfacing modules (220, 230, 240, 250) to be controlled by the therapy-support module (260) serves the purpose of emulating different interdependent medical aspects of the addressed condition CKD-MBD. The interfacing module to the patient monitor (220) corresponds to patient-side contributions to the medical condition and its therapy, the interfacing module to the clinical monitor corresponds to clinician-side contributions to the medical condition and its therapy, and the interfacing module to the drug-dose monitor corresponds to effects of separate drugs, their dosing, and combinational effects to the medical condition and its therapy. The interdependence of therapy-related contributions is addressed by the communication-coordinative therapy-support module (260).

In one aspect of the invention, the therapy-support module (260) is further configured to process therapy-related data transmitted via the interfacing modules (220, 230, 240, 250). This allows for the generation of therapy-related recommendations to be additionally transmitted by the interfacing modules (220, 230, 240, 250). Different recommendations can be generated to be transmitted by the respective interfacing modules (220, 230, 240, 250) corresponding to different therapy-related aspects that can be differently addressed, e.g. by clinician, patient, and drug-dosing. The purpose is the optimization of the patient's therapy outcome as result of recommended changes to the respective contributions.

**Fig. 3** provides a schematic depicting data exchanged between the therapy optimizer and other subsystems of the CDSS according to an embodiment of the invention.

The CDSS (310) adapted to supporting the therapy of CKD-MBD comprises the subsystems patient monitor (320), clinical monitor (340), drug-dose simulator (330), clinical data base (350) in respective data communication (321, 331, 341, 351) to the therapy optimizer system (360) via interfacing modules (362, 363, 364, 365) comprised by the therapy optimizer system (360), which in term can be provided with data communication to the therapy-support module (366).

In one first aspect of the present invention, the interfacing module (365) to the clinical database is configured to exchange data (351) related to patient health records with a clinical database (350). This allows to import therapy-related data from medical records stored in the clinical data base (350) via the interfacing module (365) for consideration of CKD-MBD therapy outcomes by the therapy-support module (366). Recommended therapy adaptions and effects of actions performed by patient and/or clinicians resulting from the recommendations in terms of patient condition can be recorded to the clinical data base (350). The medical records updated in this way, can iteratively be imported to the therapy optimizer system (360) to allow for an adaptive and continuous, "on-line", therapy optimization.

In one second additional or alternative aspect of the present invention, the interfacing module (364) to the clinical monitor is configured to exchange data (341) related to one or more of drug-dose adjustments, visit assessments, medical patient needs and one or more of clinical patient characteristics, biomarkers, drug treatments, medical warnings, support requests with a clinician-used clinical monitor (340) via the interfacing module (364) for consideration of CKD-MBD therapy outcomes by the therapy-support module (366). Therapy adaptions provided by the therapy optimizer system (360) requiring assessment and/or decision by a clinician can that way be communicated to the user of the clinical monitor (340). A clinician's recommendation to therapy and/or reactions to the patient's medical status and its development, can be fed back to the therapy optimizer system (360) to allow an adaptive clinician-side contribution to the therapy support.

In one third additional or alternative aspect of the present invention, the interfacing module (362) to the patient monitor is configured to exchange data (321) related to one or more of drug-dose adjustments, physical activity, visit assessments, questionnaire for clinical data and one or more of drug intake, diet, physical activity, patient-reported outcomes and sensor measurements with a patient-used patient monitor (320) via the interfacing module (362) for consideration of CKD-MBD therapy outcomes by the therapy-support module (366). Therapy adaptions provided by the therapy optimizer system (360) requiring execution by the patient can that way be communicated to the user of the patient monitor (320). A patient's reporting on their physiological parameters, drug adherence, physical activity and the like can be fed back to the therapy optimizer system (360) to allow an adaptive patient-side contribution to the therapy support.

In one fourth additional or alternative aspect of the present invention, the interfacing module (363) to the drug-dosing simulator is configured to exchange data (331) related to a patient's dose-response relation and one or more of drug intake, diet, biomarkers, patient's characteristics with a drug dose-response simulator (330) via the interfacing module (363) for consideration of CKD-MBD therapy outcomes by the therapy-support module (366). Physiological and drug-related aspects of the therapy provided by the therapy optimizer (360) required for the simulation of the patient's drug response can that way be communicated to the drug-response simulator (330). The results of the simulation in terms of drugs and their dosing, as well as quantity and quality for best therapy outcomes, i.e. improvement of the patient-characteristic biomarkers, in addition to variance imposed by non-drug aspects of the therapy (e.g. diet, physical activity) can be fed back to the therapy optimizer system (360) to allow an adaptive drug-response contribution to the therapy support.

Stated first to forth aspects of the invention can be employed in any combination to satisfy the needs of the underlying CDSS and to best emulate the contributions to the specific CKD-MBD therapy. The additional combination with further interfacing modules to other CDSS subsystems can be anticipated by the person skilled in the art to provide further potential for therapy improvement. Particular advantages can be found in the combination of the stated third and fourth aspects of the invention, without further configuration of the interfacing modules to a clinical monitor or a clinical database, for therapy cases, in which therapy outcomes are mainly dependent on patient behavior and drug dosing (adherence), and clinical contributions can be neglected. Additional or alternative particular advantages can be found in the combination of the stated second and third aspects of the invention, without further configuration of the interfacing modules to a drug-dosing simulator or a clinical database, for therapy cases, in which the interactions between clinician and patient, e.g. by regular checkups, are the major contributor to the therapy outcomes, and drug dosing (adherence) can be neglected. Furthermore, additional or alternative particular advantages can be found in the combination of the stated first, second and fourth aspects of the invention, without specific configuration of the interfacing modules to a patient monitor, for cases, which are not specific for a particular patient, but are mostly influenced by drug-dosing with respect to medical records; e.g. in the frame of statistical drug-response data analytics.

The interfacing modules (362, 363, 364, 365) of the therapy optimizer system (360) can be configured to exchange treatment-related data (321, 331, 341, 451) with subsystems of the CDSS. Since, generally, the subsystems are each specialized to perform individual tasks independent of their combined employment in a CDSS adapted to optimize the therapy of CKD-MBD, a data formatting and processing specifically suited to that respective task can be appropriate to be different for each subsystem. For instance, the clinical database (350) might prefer formatting the data within a clinic-specific health-record form sheet or a data format suited for improved exchange between different clinics via, e.g. a certain compressing or indexing system. For instance, the drug-dosing simulator (330) might prefer formatting the data in terms of model variables and their ranges for improved importing into the algorithmic evaluation underlying the simulation. Since the therapy module (366) of the therapy optimizer system (360) combines the therapy-related data and another data format might be better suited for its processing , it is therefore desirable to transform the data exchanged via the interfacing modules (362, 363, 364, 365). Therefore, in one aspect of the present invention, the interfacing modules (362, 363, 364, 365) are further configured to transform therapy-related data exchanged by the interfacing modules between a data format used in the processing of the therapy-support module (366) and a data format of the data transmitted to the respective interfacing modules. The transformation can include re-allocation of the data-structure parts, their combination, conditional filtering, and other methods suitable to adapt the format, in which the data is exchanged and processed.

The processing of the therapy-related data by the therapy-support module (366) of the therapy optimizer system (360) can start by aggregating the data by controlling the interfacing modules (362, 363, 364, 365) to transfer data from the CDSS subsystems. The aggregated data can then be pre-analyzed for data relevant for the therapy of CKD-MBD as preconfigured in definitions. At this pre-processing step, redundant information contained in the data transferred from different subsystems, as well as information irrelevant for the therapy of CKD-MBD, can be filtered out before being processed by the therapy-support module (366). This reduces the required processing capabilities of the module.

According to one aspect of the present invention, the treatment-related data can be analyzed within the processing of the therapy-support module (366) with respect to their sufficiency for generating therapy-related recommendations. The sufficiency analysis can be performed by matching the aggregated data to preset definitions of data required for sufficiency. Here, sufficiency for generating therapy-related recommendations can be defined by the different aspects of the therapy (e.g. biomarkers, drug dosing, diet, physical activity, etc.) and their respective representation by the aggregated data. In one instance, sufficiency can be established by a complete set of information related to the patient's medical history in terms of drug intake if the only contribution to the patient's specific CKD-MBD therapy is on the drug-dosing side. In another instance, sufficiency can be established only by a complete set of information related to the patient's physical activity and medication adherence, the clinician's prescription and the minimum requirement of the drug-response simulator to produce a recommended drug dosing. The sufficiency criterion is not necessarily limited to be established only if the amount of available therapy-related data exceed a certain degree of potentially available or required data, e.g. 60% of input data required by the drug-response simulation, but can also be established if the minimum required data could be derived from the available therapy-related data by derivation or deduction. Means of data derivation/deduction can include algorithmic analysis such as performed in the frame of artificial neural networks including approaches of deep learning, Bayesian networks, evolutionary algorithms, and others. The sufficiency analysis poses an advantageous step in preventing recommendations based on insufficient data and therefore directly contributes to the quality of the resulting therapy optimization, as well as to the efficiency of the process.

In one aspect of the present invention, the therapy-support module (366) is configured to, based on the sufficiency analysis, control the interfacing modules (362, 363, 364, 365) to request additional data-input from the CDSS subsystems, i.e. generate and transmit data-input requests via the interfacing modules (362, 363, 364, 365),. This step is preferable to be performed if the therapy-related data aggregated is analyzed to not be sufficient for generating therapy-related recommendations. The control of the therapy-support module can include identifying, from matching the aggregated data to preset definitions of data required for sufficiency, subsets of data missing for sufficiency. The process can further include identifying, which interfacing module(s) are suitable to request the subsets of data missing for sufficiency. The requests for additional data-input can contain indexes of the missing data subsets. The therapy-support module generates data-input requests considering the sufficiency analysis, missing data subsets and suitable interfacing modules. The requests can be transformed by the reformatting process of the interfacing modules to provide the request in a data format preferred by the subsystem the interfacing module is exchanging data with. The therapy-support module's controlling the interfacing modules to transmit the generated data-input requests allows for sequential improvement of the CKD-MBD therapy, particularly in cases in which initially provided therapy-related data is not sufficient for the generating of recommendations. Including a common time stamp criterion of aggregated therapy-related data for the consideration for sufficiency allows the data-input requests to be generated in case certain data sub-sets are outdated, which supports the therapy optimization to be performed in a continuous and adaptive manner. This is desirable even if the aggregated data set is complete with respect to sufficiency for a recommendation.

According to one aspect of the invention, in case the data aggregated by the therapy-support module (366) are analyzed to be sufficient, the therapy-support module (366) is further configured to generate, based on the therapy-related data (321, 331, 341, 451) transmitted to the interfacing modules (362, 363, 364, 365), therapy-related recommendations to be transmitted by the respective interfacing modules (362, 363, 364, 365). The contents of therapy-related recommendations can be identified by the aspects of the patient's specific CKD-MBD therapy in terms of the status of the therapy parameters and potential for improvement as assessed by the clinician and as results(s) of the drug-response simulation. The high influence of the patient's end on the therapy (medication adherence, diet, physical activity, etc.) specific for the medical condition CKD-MBD can make the patient a typical target of such recommendations. In one instance, depending on the patient's change in diet, the therapy-support module (366) might identify a difference between the patient's current drug dosing and the one resulting as optimal from a drug-response simulator (330) according to the change of patient's diet and other physiological parameters as obtained from a clinical data base (350). The identified difference in drug-dosing in this case would be employed by the therapy support-module (366) to generate a recommendation to the patient to be transferred via the interfacing module (362) to the patient monitor (320); preferably advising the user of the patient monitor (320) to adapt the drug dose in accordance with the format used by the patient monitor. In another instance, the aggregated therapy parameters are identified to exceed the values defined to be critical (i.e. trigger a medical warning) for the specific patient by the clinician interfacing with a clinical monitor (340). In this case the therapy-support module (366) can generate a recommendation with the content of the critical parameters and controls the interfacing module (364) to include the recommendation into the therapy-related data (341) exchanged with the clinical monitor (340); preferably advising the user of the clinical monitor (340) to check the critical value in the format used by the clinical monitor and/or request the user to appoint a checkup with the patient. Additionally, the therapy-support module (366) can control the interfacing module (365) to the clinical data base (350) to transfer generated treatment-related recommendations to the clinical data base (350) for storage and integration into the patient's health records. The generation of therapy-related recommendations by the therapy-support module (366) and their transfer to the respective CDSS subsystems by the interfacing modules (362, 363, 364, 365) allows to prompt changes for optimizing the CKD-MBD therapy specific to the patient that are relying on the system's user to be executed. The identification of recommendation content and their distribution to the interfacing modules interfacing modules (362, 363, 364, 365) by the therapy-support module (366) tackles the significant interdependence of the CKD-MBD therapy aspects by specifically addressing therapy aspects contributing to the improvement of the clinical outcome via separate modules. This way the direct contributors to the therapy can be disentangled and addressed sequentially in a manner adaptive to the dynamics of the patient's medical status.

In one aspect of the invention, the generated requests for additional data-input can be part of the therapy-related recommendations generated by the therapy-support module (366). This is preferable in situations, where the aggregated data are analyzed to be not sufficient to generate recommendations with respect to certain aspects of the CKD-MBD therapy but sufficient to generate recommendations with respect of certain other aspects. This is also preferable in situations, where the aggregated data are analyzed to be sufficient to generate recommendations but the statistical uncertainty of the underlying data and/or the resulting recommendations exceeds a preset value. A measure for the matching of the aggregated data to the requirements for a statistically precise recommendation with regards to all contributing therapy aspects, is the recommendation accuracy. The recommendation accuracy can be additionally incorporated in the sufficiency criterion, i.e. the accuracy has to exceed a required pre-set value, for generating requests for additional data-input and/or therapy-related recommendations. The recommendation accuracy can also be included as part of the generated recommendation. The inclusion of the recommendation accuracy for sufficiency and/or as part of the generated recommendations allows for an additional quality measure of the therapy optimization. Additionally, the prevention of generating of inaccurate recommendations by the therapy-support module (366) and their transfer via the interfacing modules (362, 363, 364, 365) reduces the processing and data transfer load on the system; parallel to improving the therapy outcomes. According to one aspect of the invention, the required recommendation accuracy is set according to additional accuracy-setting, e.g. user-input, data transferred to the interfacing modules (362, 363, 364, 365). This allows the pre-set recommendation accuracy to be adapted with regards to the dynamics of the optimized CKD-MBD therapy. This additionally allows the users of the CDSS's subsystems to include feedback, corresponding to the quality of the recommendations generated by the therapy-support module (366), into the data (321, 341, 351) exchanged via the interfacing modules. A person skilled in the art will acknowledge this feature's contribution to adaptive algorithmic evaluations, e.g. reinforcement learning, performed as part of the system's processing.

Since the therapy optimizer system (360) of the present invention can be implemented by software means on a computational system, one aspect of the invention defines the computer-implemented method to control the processes performed by the therapy optimizer system (360). The method comprises the steps of transmitting therapy-related data to the interfacing modules; analyzing the therapy-related data transmitted by the interfacing modules with respect to their sufficiency for producing therapy-related recommendations; generating, based on a sufficiency analysis, data-input requests to be transmitted by the respective interfacing modules; generating, based on the therapy-related data transmitted to the interfacing modules, therapy-related recommendations to be transmitted by the respective interfacing modules; controlling the interface modules to transmit respective data-input requests and/or therapy-related recommendations.

The therapy optimizer system's configuration to perform therapy optimization in an adaptive and continuous manner, as outlined in various aspects of the invention, translates to continuity and adaptiveness of the computer-implemented method; i.e., the method steps can be interchanged in order and/or iterated or omitted, so far as in accordance with logic and technical feasibility.

In one embodiment of the invention, a computer program is provided, the program comprising instructions which, when the program is executed by a computer, cause the computer to carry out the steps of the method performed by the therapy optimizer system.

In one embodiment of the invention, a computer program product is provided, the computer program product comprising instructions for performing the steps of the method performed by the therapy optimizer.

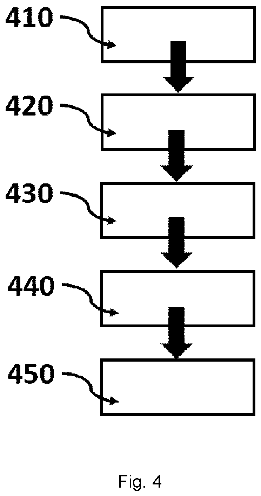

**Fig. 4** provides a flowchart depicting an example process performed by the therapy optimizer system according to the present invention.

One example process is initiated by the therapy optimizer system's interfacing module to the patient monitor receiving (410) data related to the increase of phosphate consumption by the patient's dietary changes. The therapy-support module receives the data and analyzes them to not be sufficient for generating therapy-related recommendations. Therefore, the therapy support module generates requests for additional data inputs to be transferred (420) by the interfacing modules, e.g. to request therapy-related data concerning the patient's actual drug dose from the clinical data base and to request therapy-related data concerning biomarkers from the clinical monitor. Therapy-related data received via the interfacing modules as result of the requests are combined with the initial data related to patient diet by the therapy-support module and the aggregated data is transferred (430) to the interfacing module to the drug-response simulator of the CDSS with a request generated by the therapy-support module for a set of values of CKD-MBD biomarkers considering variation on phosphate binders and related drugs. The drug-response simulator results are transmitted via the interfacing module to the therapy-support module, where based on the transmitted data, an adaption of the patient's drug dosing is employed to generate a recommendation for therapy optimization (440). The therapy-support module then controls the interfacing modules to transmit (450) the therapy-related recommendation, to the patient monitor in form of an advice to change drug dosing, to the clinical monitor in form of a notification concerning changes in the patient's CKD-MBD therapy, and to the clinical data base in form of an update to the patient's medical records.

## Claims

1. A therapy optimizer system (210) for support of therapy of diseases comprising
- interfacing modules (220, 230, 240, 250) configured for transmitting therapy-related data with one or more of a clinical data base, a clinical monitor, a dose-response simulator, a patient monitor
- a therapy-support module (260) configured to control the transmission of therapy-related data by the interfacing modules (220, 230, 240, 250), whereby the therapy-support module (260) is further configured to process the transmitted therapy-related data to generate therapy-related recommendations to be additionally transmitted by the interfacing modules (220, 230, 240, 250).

2. The therapy optimizer system (210) according to claim 1, whereby the system is configured to support the therapy of a disease selectable from the list of CKD-MBD, hypotension, diabetes, coronary heart/artery disease, asthma, obesity, cancer.

3. The therapy optimizer system (210) according to claims 1 or 2, whereby one interfacing module (250) is further configured to transmit data related to patient health records with a clinical database.

4. The therapy optimizer system (210) according to any of claims 1 to 3, whereby one interfacing module (240) is further configured to transmit data related to one or more of drug-dose adjustments, visit assessments, medical patient needs and one or more of clinical patient characteristics, biomarkers, drug treatments, medical warnings, support requests with a clinician-used clinical monitor.

5. The therapy optimizer system (210) according to any of claims 1 to 4, whereby one interfacing module (230) is further configured to transmit data related to a patient's dose-response relation and one or more of drug intake, diet, biomarkers, patient's characteristics with a drug dose-response simulator.

6. The therapy optimizer system (210) according to any of claims 1 to 5, whereby one interfacing module (220) is further configured to transmit data related to one or more of drug-dose adjustments, physical activity, visit assessments, questionnaire for clinical data and one or more of drug intake, diet, physical activity, patient-reported outcomes and sensor measurements with a patient-used patient monitor.

7. The therapy optimizer system (210) according to any of claims 1 to 6, whereby the interfacing modules (220, 230, 240, 250) are further configured to transform therapy-related data transmitted by the interfacing modules (220, 230, 240, 250) between a data format used in the processing of the therapy-support module (260) and a data format of the data transmitted to the respective interfacing modules (220, 230, 240, 250).

8. The therapy optimizer system (210) according to any of claims 1 to 7, whereby the therapy-support module (260) is further configured to analyze the therapy-related data transmitted to the interfacing modules (220, 230, 240, 250) with respect to their sufficiency for generating therapy-related recommendations.

9. The therapy optimizer system (210) according to claim 8, whereby the therapy-support module (260) is further configured to generate data-input requests to be transmitted by the respective interfacing modules (220, 230, 240, 250) if the therapy-related data transmitted to the interfacing module (220, 230, 240, 250) are analyzed to not be sufficient for generating therapy-related recommendations.

10. The therapy optimizer system (210) according to claim 8 or 9, whereby the therapy-support module (260) is further configured to generate, based on the therapy-related data transmitted to the interfacing modules (220, 230, 240, 250), therapy-related recommendations to be transmitted by the respective interfacing modules (220, 230, 240, 250) if the therapy-related data transmitted by the interfacing modules (220, 230, 240, 250) are analyzed to be sufficient for generating therapy-related recommendations.

11. The therapy optimizer system (210) according to claim 10, whereby the sufficiency criterion for the analysis of the therapy-related data transmitted to the interfacing modules (220, 230, 240, 250) is related to a pre-set recommendation accuracy and the generated therapy-related recommendations additionally include the recommendation accuracy and the generated data-input requests to be transmitted by the respective interfacing modules (220, 230, 240, 250).

12. The therapy optimizer system (210) according to claim 11, whereby the recommendation accuracy is set according to additional data transmitted to the interfacing module (220, 230, 240, 250).

13. A computer-implemented method for supporting the therapy of diseases comprising the steps
- Transmitting therapy-related data to interfacing modules (220, 230, 240, 250) configured for transmitting therapy-related data with one or more of a clinical data base, a clinical monitor, a dose-response simulator, a patient monitor
- Analyzing the therapy-related data transmitted by the interfacing modules (220, 230, 240, 250) with respect to their sufficiency for producing therapy-related recommendations
- Generating, based on the sufficiency analysis, data-input requests to be transmitted by the respective interfacing modules (220, 230, 240, 250)
- Generating, based on the therapy-related data transmitted to the interfacing modules (220, 230, 240, 250), therapy-related recommendations to be transmitted by the respective interfacing modules
- Controlling the interfacing modules (220, 230, 240, 250) to transmit respective data-input requests and/or therapy-related recommendations.

14. A computer program comprising instructions which, when the program is executed by a computer, cause the computer to carry out the steps of the method of claim 13.

15. A computer program product comprising instructions which, when the program is executed by a computer, cause the computer to carry out the steps of the method of claim 13.
